# EUROPEAN PATENT SPECIFICATION

(11) **EP 3 349 651 B1**
(45) Date of publication and mention of the grant of the patent: **11.11.2026**
(21) Application number: 16778214.3
(22) Date of filing: 14.09.2016
(51) Int. Cl.: G16H 10/40, G16H 20/60, G16H 50/20, G16H 50/30, A61B 5/08, A61B 5/00

(54) **PURE NON-INVASIVE METHOD FOR IDENTIFICATION OF ORGAN DISEASES OR IMPAIRED ORGAN FUNCTION BY INVESTIGATION OF MARKER SUBSTANCES IN EXHALED AIR STIMULATED BY INHALED MARKER SUBSTANCES**
REINES NICHTINVASIVES VERFAHREN ZUR IDENTIFIZIERUNG VON ORGANKRANKHEITEN ODER BEEINTRÄCHTIGTER ORGANFUNKTION DURCH UNTERSUCHUNG VON MARKERSUBSTANZEN IN DURCH INHALIERTE MARKERSUBSTANZEN STIMULIERTER AUSGEATMETER LUFT
PROCÉDÉ NON INVASIF PUR D'IDENTIFICATION DE MALADIES TOUCHANT DES ORGANES OU D'UNE ALTÉRATION DE LA FONCTION D'ORGANES PAR ANALYSE DE SUBSTANCES DE MARQUAGE DANS L'AIR EXPIRÉ STIMULÉ PAR DES SUBSTANCES DE MARQUAGE INHALÉES

(30) Priority: 14.09.2015 DE 102015217511; 06.11.2015 DE 102015221862
(43) Date of publication of application: 25.07.2018
(73) Proprietor: Freie Universität Berlin, 14195 Berlin (DE)
(72) Inventor: VON HAIMBERGER, Theodore, 10827 Berlin (DE); HEYNE, Anja, 14547 Beelitz-Buchholz (DE); HEYNE, Karsten, 14547 Beelitz-Buchholz (DE)
(74) Representative: Maikowski & Ninnemann Patentanwälte Partnerschaft mbB
(86) International application number: PCT/EP2016/071729
(87) International publication number: WO 2017/046183

(56) References cited:
- WO-A2-2009/147576
- J BEAUCHAMP: "Inhaled today, not gone tomorrow: pharmacokinetics and environmental exposure of volatiles in exhaled breath", JOURNAL OF BREATH RESEARCH, vol. 5, no. 3, 1 September 2011 (2011-09-01), US, pages 037103, XP055336662, ISSN: 1752-7155, DOI: 10.1088/1752-7155/5/3/037103
- ANONYMOUS: "New breath test shows possible biomarker for early-stage liver disease diagnosis", 11 August 2015 (2015-08-11), XP055323325, Retrieved from the Internet <URL:http://www.birmingham.ac.uk/news/latest/2015/08/11Aug15Newbreathtestshowspossiblebiomarkerforearly-stageliverdiseasediagnosis.aspx> [retrieved on 20161125]

## Description

The invention relates to a method for providing original data that can be used for subsequently determining the function of an organ of a living organism or for subsequently diagnosing a disease or a severity of a disease of an organ of a living organism according to the preamble of claim 1, to the use of specific substances in such a method according to the preamble of claim 8 as well as to the further medical use of such substances in a diagnostic method according to the preamble of claim 10.

Non-invasive determination of organ diseases, such as liver diseases, or impaired organ function, such as impaired liver function, is an important field of medicine and hepatology. The liver is the organ where most catalytic processes, metabolic reactions, and decomposition of toxic marker substances take place. Thus, the actual metabolic power and overall status of the liver is a crucial information for physicians treating a patient.

Over the last years several new methods were developed to measure the liver function and to identify liver diseases or impaired liver functions. The two most important methods for identification of the liver function are the so-called LiMAx test and the ICG (indiocyano green) test. Both tests are based on a marker substance administered to the patients. The LiMAx-test tracks the metabolization of the ¹³C-labelled substrate methacetin as marker substance by detecting the metabolic product ¹³CO₂, while the ICG test simply detects the clearing of ICG via the liver and kidney (cf. US 2012/0330116 A1).

The information on the liver status is limited for the ICG test, since the function of the kidney influences the clearing dynamics, and more important the slow clearing dynamics also depend on redistribution processes of ICG within the body, altering the time constant measured. Thus, the ICG test is not able to provide precise information on the liver status.

In contrast, the fast LiMAx test tracks the metabolic product of ¹³C-methacetin in real time, gaining information on the liver metabolic dynamics not influenced by redistribution processes within the body. However, the LiMAx test could be influenced by lung function in case of patients with altered lung function or severe lung diseases.

For both tests intravenous administration of the marker substance (ICG or methacetin) is optimal, and oral or intestinal administration is possible. According to current knowledge, none of these tests allows an administration of the marker substance by inhalation. One reason is the necessary high concentration of the marker substance that cannot be achieved by inhalation in case of ICG or methacetin. In addition, neither methacetin nor ICG have a measurable vapor pressure, making a direct inhalation of these substances impossible.

The LiMAx test induces metabolization of ¹³C-methacetin and thus belongs to methods using induced metabolization processes after administering a marker substance by tracking a part of the metabolization process. In case of the LiMAx test, a metabolization product is tracked to follow metabolization dynamics. This is exemplarily described in WO 2007/000145 A2, WO 2011/076803 A1, and WO 2011/076804 A2. Some further references relating to scientific literature about the LiMAx test and related liver diseases are given below:
1. Jara M, Reese T, Malinowski M, Valle E, Seehofer D, Puhl G, Neuhaus P, Pratschke J, Stockmann M; Reductions in post-hepatectomy liver failure and related mortality after implementation of the LiMAx algorithm in preoperative work-up: a single- centre analysis of 1170 hepatectomies of one or more segments; HPB (Oxford). 2015;17:651-8.
2. Bednarsch J, Jara M, Lock JF, Malinowski M, Pratschke J, Stockmann M.; Noninvasive diagnosis of chemotherapy induced liver injury by LiMAx test - two case reports and a review of the literature; BMC Res Notes. 2015;8:99
3. Malinowski M, Stary V, Lock JF, Schulz A, Jara M, Seehofer D, Gebauer B, Denecke T, Geisel D, Neuhaus P, Stockmann M.; Factors influencing hypertrophy of the left lateral liver lobe after portal vein embolization; Langenbecks Arch Surg. 2015;400:237-46.
4. Jara M, Malinowski M, Bahra M, Stockmannn M, Schulz A, Pratschke J, Puhl G.; Bovine pericardium for portal vein reconstruction in abdominal surgery: a surgical guide and first experiences in a single center; Dig Surg. 2015;32:135-41.
5. Gebhardt S, Jara M, Malinowski M, Seehofer D, Puhl G, Pratschke J, Stockmann M.; Risk Factors of Metabolic Disorders After Liver Transplantation: An Analysis of Data From Fasted Patients; Transplantation. 2015;99:1243-9.
6. Malinowski M, Geisel D, Stary V, Denecke T, Seehofer D, Jara M, Baron A, Pratschke J, Gebauer B, Stockmann M.; Portal vein embolization with plug/coils improves hepatectomy outcome; J Surg Res. 2015;194:202-11.
7. Geisel D, Lüdemann L, Fröling V, Malinowski M, Stockmann M, Baron A, Gebauer B, Seehofer D, Prasad V, Denecke T.; Imaging-based evaluation of liver function: comparison of 99mTc-mebrofenin hepatobiliary scintigraphy and Gd-EOB-DTPA- enhanced MRI; Eur Radiol. 2015;25:1384-91.
8. Hoppe S, von Loeffelholz C, Lock JF, Doecke S, Sinn BV, Rieger A, Malinowski M, Pfeiffer AF, Neuhaus P, Stockmann M.; Nonalcoholic Stectohepatits and Liver Steatosis Modify Partial Hepatectomy Recovery; J Invest Surg. 2015;28:24-31.
9. Jara M, Malinowski M, Lüttgert K, Schott E, Neuhaus P, Stockmann M.; Prognostic value of enzymatic liver function for the estimation of short-term survival of liver transplant candidates: a prospective study with the LiMAx test; Transpl Int. 2015;28:52-8.
10. Jara M, Bednarsch J, Valle E, Lock JF, Malinowski M, Schulz A, Seehofer D, Jung T, Stockmann M.; Reliable assessment of liver function using LiMAx; J Surg Res. 2015;193:184-9.
11. Faber W, Sharafi S, Stockmann M, Dennecke T, Bahra M, Klein F, Malinowski MB, Schott E, Neuhaus P, Seehofer D.; Patient age and extent of liver resection influence outcome of liver resection for hepatocellular carcinoma in non-cirrhotic liver; Hepatogastroenterology. 2014;61:1925-30.
12. Faber W, Stockmann M, Kruschke JE, Denecke T, Bahra M, Seehofer D.; Implication of microscopic and macroscopic vascular invasion for liver resection in patients with hepatocellular carcinoma; Dig Surg. 2014;31:204-9.
13. Kiefer S, Schafer M, Bransch M, Brimmers P, Bartolomé D, Baños J, Orr J, Jones D, Jara M, Stockmann M.; A novel personal health system with integrated decision support and guidance for the management of chronic liver disease; Stud Health Technol Inform. 2014;205:83-7.
14. Jara M, Bednarsch J, Malinowski M, Lüttgert K, Orr J, Puhl G, Seehofer D, Neuhaus P, Stockmann M.; Predictors of quality of life in patients evaluated for liver transplantation; Clin Transplant. 2014;28:1331-8.
15. Malinowski M, Jara M, Lüttgert K, OrrJ, Lock JF, Schott E, Stockmann M.; Enzymatic Liver Function Capacity Correlates with Disease Severity of Patients with Liver Cirrhosis: A Study with the LiMAx Test; Dig Dis Sci. 2014;59:2983-91.
16. Brinkhaus G, Lock JF, Malinowski M, Denecke T, Neuhaus P, Hamm B, Gebauer B, Stockmann M.; CT-Guided High-Dose-Rate Brachytherapy of Liver Tumours Does Not Impair Hepatic Function and Shows High Overall Safety and Favourable Survival Rates; Ann Surg Oncol. 2014;21:4284-92.
17. Jara M, Bednarsch J, Lock JF, Malinowski M, Schulz A, Seehofer D, Stockmann M.; Enhancing safety in liver surgery using a new diagnostic tool for evaluation of actual liver function capacity - The LiMAx test; Dtsch Med Wochenschr. 2014;139:387-91.
18. Faber W, Stockmann M, Schirmer C, Möllerarnd A, Denecke T, Bahra M, Klein F, Schott E, Neuhaus P, Seehofer D.; Significant impact of patient age on outcome after liver resection for HCC in cirrhosis; Eur J Surg Oncol. 2014;40:208-13.
19. Geisel D, Malinowski M, Powerski MJ, Wüstefeld J, Heller V, Denecke T, Stockmann M, Gebauer B.; Improved Hypertrophy of Future Remnant Liver after Portal Vein Embolization with Plugs, Coils and Particles; Cardiovasc Intervent Radiol. 2014;37:1251-8.
20. Kaffarnik MF, Lock JF, Vetter H, Ahmadi N, Lojewski C, Malinowski M, Neuhaus P, Stockmann M.; Early diagnosis of sepsis-related hepatic dysfunction and its prognostic impact on survival: a prospective study with the LiMAx test; Crit Care. 2013;17:R259.
21. Holzhütter HG, Lock JF, Taheri P, Bulik S, Goede A, Stockmann M.; Assessment of hepatic detoxification activity: proposal of an improved variant of the (13)c- methacetin breath test; PLoS One. 2013;8:e70780.
22. Döcke S, Lock JF, Birkenfeld AL, Hoppe S, Lieske S, Rieger A, Raschzok N, Sauer IM, Florian S, Osterhoff MA, Heller R, Herrmann K, Lindenmüller S, Horn P, Bauer M, Weickert MO, Neuhaus P, Stockmann M, Möhlig M, Pfeiffer AF, von Loeffelholz C.; Elevated hepatic chemerin gene expression in progressed human non-alcoholic fatty liver disease; Eur J Endocrinol. 2013;169:547-57.
23. Geisel D, Lüdemann L, Wagner C, Stelter L, Grieser C, Malinowski M, Stockmann M, Seehofer D, Hamm B, Gebauer B, Denecke T.; Evaluation of gadolinium-EOB-DTPA uptake after portal vein embolization: value of an increased flip angle; Acta Radiol. 2014;55:149-54.
24. Strücker B, Stockmann M, Denecke T, Neuhaus P, Seehofer D.; Intraoperative Placement of External Biliary Drains for Prevention and Treatment of Bile Leaks After Extended Liver Resection Without Bilioenteric Anastomosis; World J Surg.2013;37:2629-34.
25. Lock JF, Kotobi AN, Malinowski M, Schulz A, Jara M, Neuhaus P, Stockmann M.; Predicting the prognosis in acute liver failure: results from a retrospective pilot study using the LiMAx test; Ann Hepatol. 2013;12:556-62.
26. Geisel D, Lüdemann L, Keuchel T, Malinowski M, Seehofer D, Stockmann M, Hamm B, Gebauer B, Denecke T.; Increase in left liver lobe function after preoperative right portal vein embolisation assessed with gadolinium-EOB-DTPA MRI; Eur Radiol. 2013;23:2555-60.35. Stockmann M, Lock JF; How Far Is the Development of 13C-Liver-Function Breath Tests? Dig Dis Sci. 2013;58:1804-5.
27. Lederer A, It Seehofer D, Schirmeier A, Levasseur S, Stockmann M, Nüssler AK, Menger MD, Neuhaus P, Rayes N.; Postoperative bile leakage inhibits liver regeneration after 70% hepatectomy in rats; J Invest Surg. 2013;26:36-45.
28. Faber W, It Sharafi S, Stockmann M, Denecke T, Sinn B, Puhl G, Bahra M, Malinowski MB, Neuhaus P, Seehofer D.; Long-term results of liver resection for hepatocellular carcinoma in noncirrhotic liver; Surgery. 2013;153:510-7.

WO 2012/140213 A2 describes a method in which a marker substance is administrated, wherein the metabolization products are detected in real time.

WO2009/147576 (A2) discloses a system comprising multiple inhalation markers containers, which can be used for sequential disease characterization.

It is an object of the present invention to provide a method that can be used in organ function diagnostics or in (quantitative) diagnostics of organ diseases, wherein the method allows for totally non-invasive administration of a marker substance to a living organism.

It was surprisingly found that inhalation of a marker substance is a suited way of administration, if not a metabolization product of the marker substance, but the marker substance itself is detected afterwards in air exhaled by the living organism. Specifically, a reduction of the concentration of the marker substance in the exhaled air can be directly linked to a metabolization of a part of the marker substance by the living organism in a specific organ. Thereby, the kind of marker substance is the decisive factor for the organ, the function of which is to be determined.

Specifically, the object is solved by a method for providing original data that can be used for subsequently determining the function of an organ of a living organism or for subsequently diagnosing a disease or a severity of a disease of an organ of a living organism. This method is characterized by the following steps:
a) administering a marker substance to a living organism by inhalation, wherein the marker substance has a vapor pressure above 0.01 mmHg at 37 °C,
b) determining a concentration of the marker substance in exhaled air which is exhaled by the living organism at a first time point,
c) determining the concentration of the marker substance in the exhaled air which is exhaled by the living organism at a second time point after the first time point,
d) determining a difference between the concentration of the marker substance determined at the first time point and the concentration of the marker substance determined at the second time point.

The living organism can be, e.g., a rodent or a mammal, such as a human.

Inhalation is a highly non-invasive method to administer a marker substance and guarantees that the lung condition of the living organism is reflected in the uptake/inhalation and release/exhalation of the marker substance.

The method steps can be performed in the sequence indicated above or in any other sequence. If the method steps are performed in the sequence indicated above, the first time point is a time point after inhalation of the marker substance. E.g., it can be a time point directly after inhalation of the marker substance. In this circumstance, "directly" means within less than 1 minute, in particular less than 45 seconds, in particular less than 30 seconds, in particular less than 15 seconds after the end of inhalation of the marker substance.

Such a method step sequence is particularly suited for providing original data to be used in liver function diagnostics or in diagnosing liver diseases.

According to the invention, the second time point is always after the first time point. The time difference between the first time point and the second time point can be, e.g., between from 30 seconds to 2 days. At least two measurements have to be taken, but increasing the number of data points (measurements) increases the certainty of the metabolization dynamics observed indirectly by a decrease of the concentration of the marker substance in the exhaled air.

Suitable time points for a measurement (and therewith also suited differences between the first time point and the second time point) are 30 seconds, 1 minute, 2 minutes, 3 minutes, 5 minutes, 7 minutes, 10 minutes, 20 minutes, 30 minutes, 40 minutes, 50 minutes, 70 minutes, 100 minutes, 200 minutes, 300 minutes, 400 minutes, 500 minutes, 700 minutes, 1000 minutes, 2000 minutes, or 2800 minutes after beginning of inhalation, before the inhalation, or after the end of inhalation.

Information on the metabolization dynamics of the marker substance can be generally gathered from measurements before inhaling the enriched air, while inhaling the enriched air, directly after inhaling the enriched air, and after inhalation of the enriched air at different time points.

In an embodiment, step a) is performed between steps b) and c). Thus, in this embodiment, the first measurement (at the first time point) takes place before the marker substance is inhaled. This means that the first measurement reflects the situation of the living organism before inhalation of the marker substance. Afterwards, the marker substance is inhaled.

According to the invention, then the concentration of the marker substance is again determined (at the second time point) so that afterwards the difference between the marker substance concentration before inhalation and after inhalation can be calculated. Such a method step sequence is particularly suited for providing original data to be used in lung function diagnostics or in diagnosing lung diseases. In an embodiment, the marker substance is used in its natural abundance, in a non-radioactive isotopically labelled form (e.g., labelled by ¹³C, ²H and/or ¹⁵N), or in a mixture of both. The vapor enriched with the marker substance can be generated by a vaporizer, directly from a solution, or by mixing different gases (one with the marker substance).

The enriched vapor can be inhaled via a filled breath bag, an open or closed system with a breath mask connected to the vapor reservoir, or other systems enriching the air with the marker substance vapor.

The marker substance concentration can be measured at any time, namely before inhaling the enriched air, while inhaling the enriched air, directly after inhaling the enriched air, and after inhalation of the enriched air.

Information on the lung properties and the ability for marker substance uptake via the lungs are taken from the measurements before inhaling the enriched air, while inhaling the enriched air, and/or directly after inhaling the enriched air.

Using isotopically labelled marker substances for inhalation allows for separation of naturally occurring marker substance levels and inhalation induced marker substance levels. In case of some marker substances there is a naturally occurring marker substance level in the blood (i.e., a natural abundance), varying for example with the time of the year. Isotopically labelled marker substances can be used to distinguish between the natural occurrence and the induced occurrence of the marker substance in the blood.

In an embodiment, the organ is at least one from the group consisting of liver, kidney, spleen, and lung. Thereby, the liver is particularly suited as organ, the function of which is to be tested. In addition, the lung is also particularly suited as organ, the function which is to be tested. Liver and lung are a particularly suited combination of organs.

In an embodiment, the marker substance is a volatile organic compound (VOC). Such volatile organic compounds are small organic molecules having a comparatively low boiling point. They occur - in different compositions - almost everywhere and are (in comparatively low doses) inhaled and again expired by all organisms. Thereby, a metabolism of these compounds can occur within the subject.

In an embodiment, the marker substance has a vapor pressure above 0.02 mmHg, in particular above 0.03 mmHg, in particular above 0.05 mmHg, in particular above 0.1 mmHg, in particular above 0.2 mmHg, in particular above 0.3 mmHg, in particular above 0.5 mmHg, and especially above 1 mmHg (always at a temperature of 37 °C).

In an embodiment, the marker substance is metabolized by the living organism. Then, the reduction of the marker substance concentration in the exhaled air can be directly related to the metabolization dynamics of the marker substance in the respective organ of the living organism.

The following substances are generally suited as marker substances. They are ordered according to their mass over charge ratio m/z (without H⁺):
m/z = 60: carbonyl sulfide, dimethylsilane, acetic acid, propanol
m/z = 108: bis(methylthio)methane, 3-mercaptopropane-1,2,-diol
m/z = 121: cysteine
m/z = 168: selenocysteine
m/z = 114: octane, furan-2-ylmethanethiol
m/z = 80: 1,2-diazine (pyridazine), 1,3-diazine (pyrimidine), 1,4-diazine (pyrazine)
m/z = 136: limonene, α-pinene, β-pinene, γ-pinene
m/z = 86: 2-pentanone, hexane
m/z = 156: 4-hydroxynonenal
m/z = 128: nonane, naphthalene

Since not all of the before-mentioned marker substances can be equally well inhaled by living organism without causing undesired side effects, the marker substance is, in an embodiment, chosen from the group consisting of octane, furan-2-ylmethanethiol, 1,2-diazine, 1,3-diazine, 1,4-diazine, a terpene, 2-pentanone, hexane and 4-hydroxynonenal. Combinations of these substances are possible.

In an embodiment, the marker substance is at least one diazine, namely 1,2-diazine, 1,3-diazine and/or 1,4-diazine.

In an embodiment, the marker substance is a terpene. In another embodiment, the terpene is chosen from the group consisting of limonene, α-pinene, β-pinene and γ-pinene. Limonene is particularly suited as marker substance.

In an aspect, the invention also relates to a method for determining the function of an organ of a living organism having the features as explained above. Thereby, the method encompasses an additional step of determining the function of the organ based on the concentration difference determined in step d). In an embodiment, the method also comprises the step of reporting the function of the organ. In an embodiment, the method comprises determining the health status of the living organism with respect to and based on the determined organ function. In an embodiment, the method also comprises the step of reporting the health status of the living organism.

In an aspect, the invention also relates to a method for diagnosing a disease or for diagnosing a severity of a disease of an organ of a living organism having the features explained above. Thereby, the method encompasses an additional step of making a diagnosis based on the concentration difference determined in step d).

In an aspect, the invention also relates to the use of at least one substance chosen from the group consisting of octane, furan-2-ylmethanethiol, 1,2-diazine, 1,3-diazine, 1,4-diazine, a terpene, 2-pentanone, hexane and 4-hydroxynonenal as marker substance to be administered to a living organism by inhalation in a method for providing original data that can be used for subsequently determining the function of an organ of the living organism or for subsequently diagnosing a disease or a severity of a disease of an organ of the living organism.

In an embodiment, the terpene is at least one of the group consisting of limonene, α-pinene, β-pinene and γ-pinene.

In an aspect, the invention also relates to the further medical use of a substance chosen from the group consisting of octane, furan-2-ylmethanethiol, 1,2-diazine, 1,3-diazine, 1,4-diazine, a terpene, 2-pentanone, hexane and 4-hydroxynonenal, namely for use as marker substance to be administered to a living organism by inhalation in a diagnostic method for determining the function of an organ of the living organism or for diagnosing a disease or a severity of a disease of an organ of the living organism.

In an embodiment, the terpene is at least one of the group consisting of limonene, α-pinene, β-pinene and γ-pinene.

All embodiments explained with respect to the described methods, uses and further medical uses can be combined in any desired way. Thereby, embodiments of the described methods can be transferred to the respective other method, described use and further medical use of the marker substances and vice versa.

Aspects of the instant invention will now be explained with respect to exemplary embodiments and accompanying Figures. In the Figures:
- Figure 1A: shows the results of proton-transfer reaction mass spectrometry (PTR-MS) with respect to compounds having an m/z value of 137 of exhaled breath of individuals having divers health conditions or nutritional states;
- Figure 1B: shows the results of PTR-MS of exhaled breath with respect to compounds having an m/z value of 137 of exhaled breath of the same individuals as in Figure 1A in dependence on the liver power (expressed as LiMAx value) that has been determined for these individuals independently on the PTR-MS measurement;
- Figure 2A: shows the results of proton-transfer reaction mass spectrometry (PTR-MS) with respect to compounds having an m/z value of 61 of exhaled breath of individuals having divers health conditions or nutritional states;
- Figure 2B: shows the results of PTR-MS of exhaled breath with respect to compounds having an m/z value of 61 of exhaled breath of the same individuals as in Figure 2A in dependence on the liver power (expressed as LiMAx value) that has been determined for these individuals independently on the PTR-MS measurement;
- Figure 3A: shows the results of proton-transfer reaction mass spectrometry (PTR-MS) with respect to compounds having an m/z value of 109 of exhaled breath of individuals having divers health conditions or nutritional states;
- Figure 3B: shows the results of PTR-MS of exhaled breath with respect to compounds having an m/z value of 109 of exhaled breath of the same individuals as in Figure 3A in dependence on the liver power (expressed as LiMAx value) that has been determined for these individuals independently on the PTR-MS measurement;
- Figure 4A: shows the results of proton-transfer reaction mass spectrometry (PTR-MS) with respect to cysteine having an m/z value of 122 of exhaled breath of individuals having divers health conditions or nutritional states;
- Figure 4B: shows the results of PTR-MS of exhaled breath with respect to cysteine having an m/z value of 122 of exhaled breath of the same individuals as in Figure 4A in dependence on the liver power (expressed as LiMAx value) that has been determined for these individuals independently on the PTR-MS measurement;
- Figure 5A: shows the results of proton-transfer reaction mass spectrometry (PTR-MS) with respect to selenocysteine having an m/z value of 169 of exhaled breath of individuals having divers health conditions or nutritional states;
- Figure 5B: shows the results of PTR-MS of exhaled breath with respect to selenocysteine having an m/z value of 169 of exhaled breath of the same individuals as in Figure 5A in dependence on the liver power (expressed as LiMAx value) that has been determined for these individuals independently on the PTR-MS measurement;
- Figure 6A: shows the results of proton-transfer reaction mass spectrometry (PTR-MS) with respect to compounds having an m/z value of 115 of exhaled breath of individuals having divers health conditions or nutritional states;
- Figure 6B: shows the results of PTR-MS of exhaled breath with respect to compounds having an m/z value of 115 of exhaled breath of the same individuals as in Figure 6A in dependence on the liver power (expressed as LiMAx value) that has been determined for these individuals independently on the PTR-MS measurement;
- Figure 7A: shows the results of proton-transfer reaction mass spectrometry (PTR-MS) with respect to compounds having an m/z value of 129 of exhaled breath of individuals having divers health conditions or nutritional states;
- Figure 7B: shows the results of PTR-MS of exhaled breath with respect to compounds having an m/z value of 129 of exhaled breath of the same individuals as in Figure 7A in dependence on the liver power (expressed as LiMAx value) that has been determined for these individuals independently on the PTR-MS measurement;
- Figure 8A: shows the results of proton-transfer reaction mass spectrometry (PTR-MS) with respect to compounds having an m/z value of 81 of exhaled breath of individuals having divers health conditions or nutritional states;
- Figure 8B: shows the results of PTR-MS of exhaled breath with respect to compounds having an m/z value of 81 of exhaled breath of the same individuals as in Figure 8A in dependence on the liver power (expressed as LiMAx value) that has been determined for these individuals independently on the PTR-MS measurement;
- Figure 9A: shows the results of proton-transfer reaction mass spectrometry (PTR-MS) with respect to compounds having an m/z value of 87 of exhaled breath of individuals having divers health conditions or nutritional states;
- Figure 9B: shows the results of PTR-MS of exhaled breath with respect to compounds having an m/z value of 87 of exhaled breath of the same individuals as in Figure 9A in dependence on the liver power (expressed as LiMAx value) that has been determined for these individuals independently on the PTR-MS measurement;
- Figure 10A: shows the results of proton-transfer reaction mass spectrometry (PTR-MS) with respect to 4-hydroxynonenal having an m/z value of 157 of exhaled breath of individuals having divers health conditions or nutritional states; and
- Figure 10B: shows the results of PTR-MS of exhaled breath with respect to 4-hydroxynonenal having an m/z value of 157 of exhaled breath of the same individuals as in Figure 10A in dependence on the liver power (expressed as LiMAx value) that has been determined for these individuals independently on the PTR-MS measurement.

### Exemplary embodiments

Suited marker substances were identified by re-evaluating the experimental data of the dissertation "Analysis of breath allows for non-invasive identification and quantification of diseases and metabolic dysfunction" of Suha Adel Al-Ani that is freely available under the following internet address:
www.diss.fu-berlin.de/diss/receive/FUDISS_thesis_000000100227

Further details of the concrete experimental work that has been done to obtain the data explained in the following can be found in chapter 4 of this dissertation. This dissertation, in particular chapter 4 regarding the experimental work, chapter 3 regarding details of DOB kinetics, and the graphically depicted results of chapter 5, is hereby incorporated by reference. Briefly, the exhaled breath of healthy individuals belonging to two groups of different nutritional states (namely based on a normal diet on the one hand and based on a vegan diet on the other hand) as well as of patients suffering from a liver disease has been measured by proton-transfer reaction mass spectrometry (PTR-MS) for quantitatively identifying different volatile organic compounds (VOCs) in the measured exhaled breath.

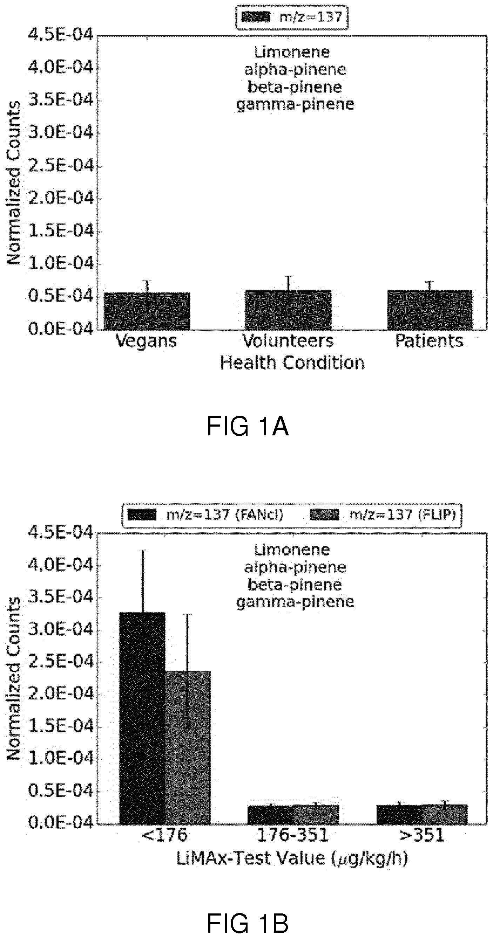

Figure 1A shows the results of according PTR-MS measurements regarding limonene and different pinenes as marker substances that are typically inhaled from the surrounding to identify liver diseases or impaired liver function. The concentration of limonene, α-pinene, β-pinene and γ-pinene (all having an m/z ratio of 137 including an additional H⁺; their mass without a proton is 136 au) is almost identical for all three groups of individuals tested (taking into account the error bars).

In addition, the liver power of the same individuals was tested by determining the LiMAx value on the basis of a breath test after ¹³C-methacetin administration. Thereby, the LiMAx value was calculated according to the following formula: $LiMAx = \frac{{DOB}_{max} ⋅ {R}_{PDB} ⋅ {P}_{{CO}_{2}} ⋅ M}{BW} ,$ wherein
the unit of the LiMAx value is (µg/kg)/h,
- DOBₘₐₓ: denotes the maximum value of the DOB (delta over baseline) kinetics,
- R_{PDB}: is the Pee Dee Belemnite standard and is 0.011237,
- P_{CO2}: denotes the CO₂ production rate that is to be calculated by (300 mmol/h)*BSA, wherein BSA means body surface area; it is indicated in m² and is calculated according to the Du Bois formula: BSA = 0.007184 * W^{0.425} * H^{0.725}, wherein W is the weight in kg and H is the height in cm of the respective individual;
- M: is the molar mass of ¹³C-methacetin (166.19 g/mol),
- BW: is the body weight of the individual in kg.

Figure 1B shows the results of PTR-MS measurements in dependence on an according determination of the LiMAx value. It can be seen from Figure 1B that the concentration of limonene, α-pinene, β-pinene and γ-pinene is significantly increased in the expiratory air of patients that have a strongly impaired liver function represented by a LiMAx value of below 176 (both bars on the left) as compared to patients with only slightly impaired liver function represented by a LiMAx value of 176 to 351 (both bars in the middle) or individuals with normal liver function represented by a LiMAx value of above 351 (both bars on the right) that were erroneously grouped as patients suffering from a liver disease but that have in fact no decreased liver function. Thereby, the LiMAx value has been determined by two independent devices, namely by a modified non-dispersive isotope-selective infrared spectrometer of Fischer Analysen Instrumente GmbH (black bars, abbreviated by FANci or FANci2-db16) or by a Flow-through Fast Liver Investigation Packet available from Humedics GmbH (grey bars, abbreviated by FLIP).

The modified non-dispersive isotope-selective infrared spectrometer FANci2-db16 has a frequency of approximately 1/min. It was used to draw and analyze breath samples. This spectrometer measures the ¹³CO₂ to ¹²CO₂ ratio. As a light source, a black body radiator is used. Two detection chambers are filled with ¹³CO₂ or ¹²CO₂, respectively, wherein a microphone is present in each detection chamber. Between the light source and the detection chamber there is a chopper to modulate the IR radiation. A measuring chamber is filled with the gas to be tested. The molecules in the detection chambers absorb the modulated IR radiation and convert it to thermal energy. The so-modulated density fluctuations cause sound waves, and each is measured with a microphone.

The disadvantage of the device is that it is very sensitive to vibrations and to temperature changes. Also, the breath cannot be measured when flowing so that it is instead kept stationary in an aluminum bag. In standard mode, the breath is collected in a bag and the bag is connected to the device, then it pumps the exhaled air into the measuring chamber. During exhaling air in to the bag, it is important to make sure that only the alveolar air is used. The air that does not reach the alveoli has the CO₂ content of the inspired air. It would distort the measured values. With this measuring method an accuracy of ±2 DOB can be achieved according to the manufacturer, but it does not provide absolute values for exhaled CO₂ volumes.

The FLIP device can measure the ¹³CO₂ to ¹²CO₂ ratio in exhaled breath. The ultra-sensitive laser spectroscopy system of the FLIP device can quickly and reliably determine the capacity of the liver function. The FLIP/LiMAx system greatly improves the surgical intervention planning. The laser based FLIP device detects a metabolic product (¹³CO₂) of the enzymatic conversion of the drug methacetin in the liver in the exhaled air. ¹³CO₂ is stable, non-radioactive and detected by the unique sensors in the device even at extremely low concentrations (100 ppb) in every single breath.

The FLIP device measures in real-time a continuous flow of air. It has been developed in cooperation with medical professionals and is adapted to various clinical situations. The FLIP has unified the mobility, the usability and the practicality. It has been used successfully in various intensive care units, emergency rooms, operating theatres and outpatient stations.

The data shown in Figures 1A and 1B was interpreted in the above-mentioned dissertation such that the according substances were held to be no good biomarkers, since the amount of inhaled marker substance was typically not known, and thus the reference was missing.

However, it turned out that this statement is a misinterpretation of the data. In contrast to this statement, limonene is a well suited marker substance within the context of the present invention. Limonene has a low vapor pressure and a pleasant odor.

After inhalation of air with marker substance vapor (marker substance gas) the exhaled marker substance concentration is measured. This provides useful information on the lung status, when the concentration of the marker substance gas is known, because this provides direct information on the exchange rate of the lung.

After some time after the inhalation, the concentration of the marker substance in the exhaled air is measured again. A reduction in concentration (in particular the course of concentration over time) of the marker substance directly reflects the metabolization of the marker substance and thus its decomposition within the living organism.

The marker substances referred to in Figures 1A and 1B are clearly decomposed slower in patients with impaired liver function. It was reported that limonene is metabolized by enzymes of the Cytochrome P450 family. [Mizayawa, M. et al. The American Society for Pharmacology and Experimental Therapeutics, Vol. 30, No. 5, (2002), 602-607] Thus, the higher concentration of the marker substance in the exhaled air in case of patients suffering from lung disease provides direct qualitative or quantitative information on the impaired liver function. Hence, measurement of exhaled marker substance concentration provides a fast test for severe liver diseases or impaired liver function.

Mizayawa et al. also reported that limonene is known to have chemopreventive activity, and is metabolized in human liver cells by CYP 2C9 and CYP 2C19 to carveol and perillyl alcohol. Other enzymes like CYP 2C8, 2C18, and 3A4 could also play a role in this metabolization.

Michael N. Gold reported in Environmental Health Perspectives, Vol. 105, Supplement 4, 1997, pages 977-979 that "Monoterpenes such as limonene and perillyl alcohol have been shown to prevent mammary, liver, lung, and other cancers".

The use of limonene as marker substances to detect liver diseases or an impaired liver function thus exhibits the additional advantage of preventing mammary, liver, lung and other cancers. Moreover, the liver metabolization product of limonene, perillyl alcohol, also prevents liver and other cancers.

In summary, by using limonene as marker substance, a natural product is used as marker substance to detect liver diseases or an impaired liver function. Limonene itself has a preventive influence on liver and other cancers. Moreover, limonene can be easily inhaled, thus allowing its use in a purely non-invasive method. Limonene is also comfortable for the patients, because of its pleasant odor. Furthermore, it provides direct information on the lung function by detecting the blood concentration change upon inhalation of the marker substance.

In contrast to prior art methods, in this exemplary embodiment limonene is administered as marker substance by inhalation. Afterwards, its metabolization via enzymes of the Cytochrome P450 enzyme family is followed by detecting the level of the administered marker substance in the exhaled air (not of the metabolized product).

Besides limonene and/or the pinenes referred to in the first exemplary embodiment, other marker substances can be used for the described methods. These marker substances are generally characterized by two properties that need to be fulfilled. First property: Comparison of the marker substance concentrations of the three groups of vegan persons, volunteers, and patients (cf. Figure 1A) shows no significant difference (within the error bars, i.e. taking the error bars into account). Second property: Comparison of the marker substance concentrations of three different liver function groups represented by three different ranges of the LiMAx value (cf. Figure 1B) shows differences (within the error bars, i.e. taking the error bars into account).

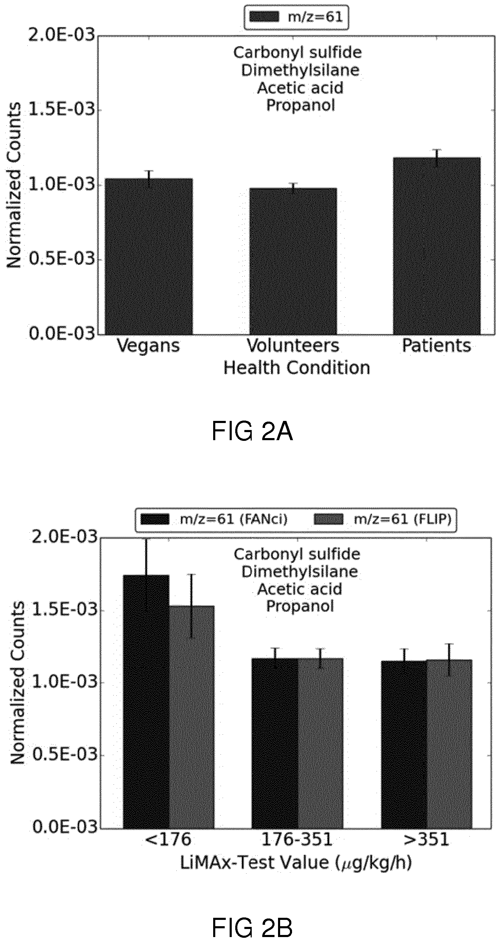

By applying these criteria to experimental data that has been previously obtained and already analyzed under a different point of view, more suited marker substances for determining the function of an organ of a living organism or for subsequently diagnosing a disease or a severity of a disease of an organ of a living organism were identified. The according data is shown in Figures 2A to 10B.

Thereby, all Figures indicate the m/z ratio of the substances identified in exhaled breath by considering an additional proton applied to the substances during PTR-MS for ionization purposes.

The results shown in Figures 2A, 3A, 4A, 5A, 6A, 7A, 8A, 9A, and 10A has been obtained in the same way as in case of Figure 1A. The results shown in Figures 2B, 3B, 4B, 5B, 6B, 7B, 8B, 9B, and 10B has been obtained in the same way as in case of Figure 1B.

Figures 2A and 2B indicate that carbonyl sulfide, dimethylsilane, acetic acid and/or propanol (having an m/z ratio of 61) are suited markers for determining the function of an organ of a living organism or for subsequently diagnosing a disease or a severity of a disease of an organ of a living organism.

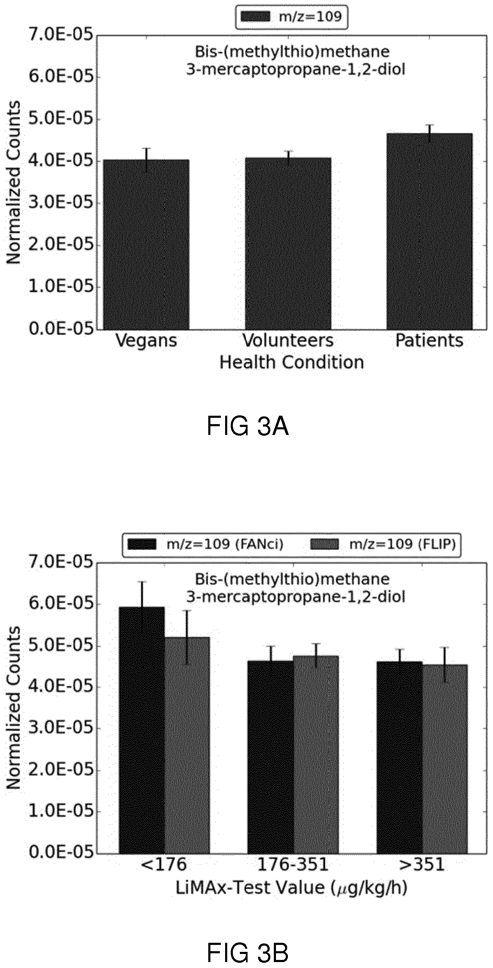

Figures 3A and 3B indicate that bis(methylthio)methane and/or 3-mercaptopropane-1,2,-diol (having an m/z ratio of 109) are suited markers for determining the function of an organ of a living organism or for subsequently diagnosing a disease or a severity of a disease of an organ of a living organism.

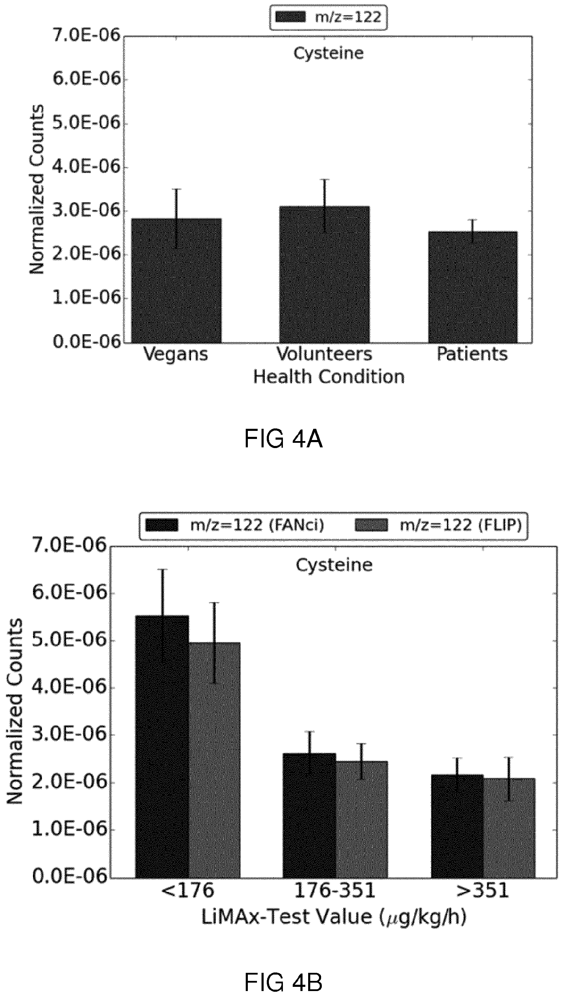

Figures 4A and 4B indicate that cysteine (having an m/z ratio of 122) is a suited marker for determining the function of an organ of a living organism or for subsequently diagnosing a disease or a severity of a disease of an organ of a living organism.

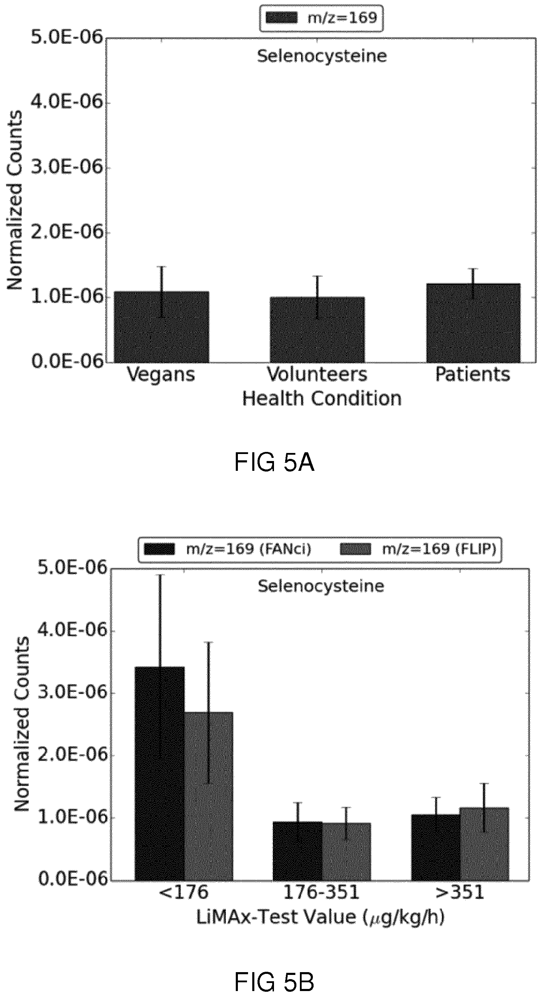

Figures 5A and 5B indicate that selenocysteine (having an m/z ratio of 169) is a suited marker for determining the function of an organ of a living organism or for subsequently diagnosing a disease or a severity of a disease of an organ of a living organism.

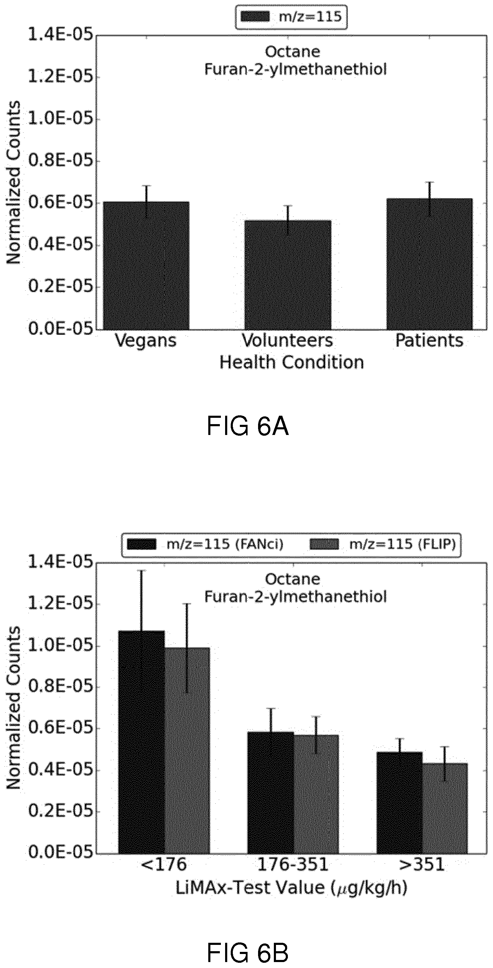

Figures 6A and 6B indicate that octane and/or furan-2-ylmethanethiol (having an m/z ratio of 115) are suited markers for determining the function of an organ of a living organism or for subsequently diagnosing a disease or a severity of a disease of an organ of a living organism.

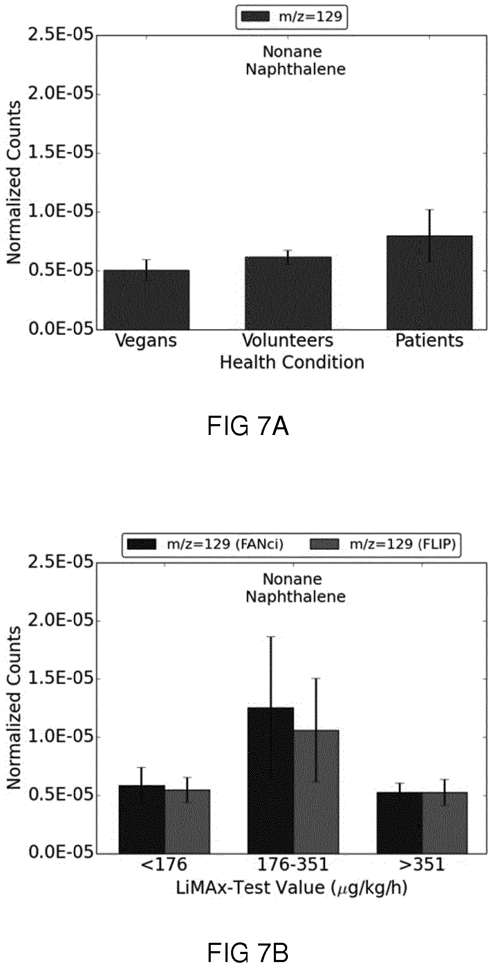

Figures 7A and 7B indicate that nonane and/or naphthalene (having an m/z ratio of 129) are suited markers for determining the function of an organ of a living organism or for subsequently diagnosing a disease or a severity of a disease of an organ of a living organism.

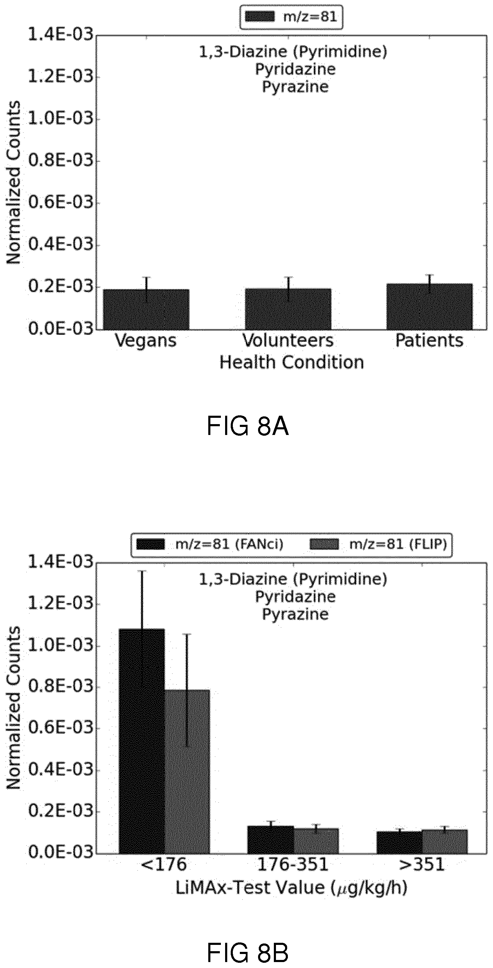

Figures 8A and 8B indicate that 1,2-diazine (pyridazine), 1,3-diazine (pyrimidine) and/or 1,4-diazine (pyrazine) (having an m/z ratio of 81) are suited markers for determining the function of an organ of a living organism or for subsequently diagnosing a disease or a severity of a disease of an organ of a living organism.

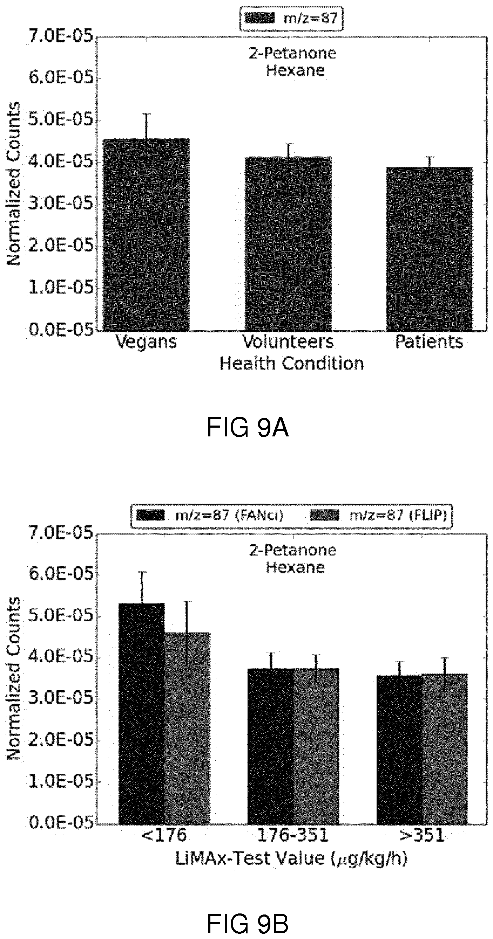

Figures 9A and 9B indicate that 2-pentanone and/or hexane (having an m/z ratio of 87) are suited markers for determining the function of an organ of a living organism or for subsequently diagnosing a disease or a severity of a disease of an organ of a living organism.

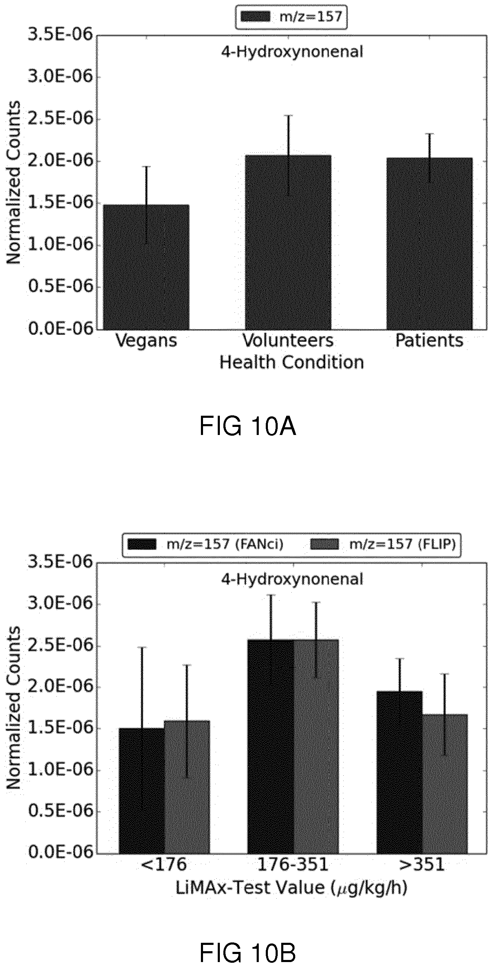

Figures 10A and 10B indicate that 4-hydroxynonenal (having an m/z ratio of 157) is a suited marker for determining the function of an organ of a living organism or for subsequently diagnosing a disease or a severity of a disease of an organ of a living organism.

## Claims

1. Method for providing original data that can be used for subsequently quantitative determining the function of an organ of a living organism or for subsequently diagnosing a severity of a disease of an organ of a living organism, the method comprising the following steps:
a) administering a marker substance to a living organism by inhalation, wherein the marker substance has a vapor pressure above 0.01 mmHg at 37 °C and is at least one substance from the group consisting of octane, furan-2-ylmethanethiol, 1,2-diazine, 1,3-diazine, 1,4-diazine, a terpene, 2-pentanone, and 4-hydroxynonenal,
b) determining a concentration of the marker substance in exhaled air which is exhaled by the living organism at a first time point, wherein the first time point is less than 30 seconds after the end of inhalation of the marker substance,
c) determining the concentration of the marker substance in the exhaled air which is exhaled by the living organism at a second time point after the first time point, wherein a difference between the first time point and the second time point is between 1 minute and 30 minutes,
d) determining a difference between the concentration of the marker substance determined at the first time point and the concentration of the marker substance determined at the second time point,
e) indirectly observing a metabolization dynamics of the marker substance in at least one of the liver, the kidney, and the spleen by a decrease of the concentration of the marker substance in the exhaled air between the first time point and the second time point.

2. Method according to claim 1, **characterized in that** the terpene is at least one of the group consisting of limonene, α-pinene, β-pinene and γ-pinene.

3. Use of at least one substance chosen from the group consisting of octane, furan-2-ylmethanethiol, 1,2-diazine, 1,3-diazine, 1,4-diazine, a terpene, 2-pentanone, and 4-hydroxynonenal as marker substance to be administered to a living organism by inhalation in a method for providing original data that can be used i) for subsequently quantitative determining the function of at least one of the liver, the kidney, and the spleen of the living organism or ii) for subsequently diagnosing a severity of a disease of at least one of the liver, the kidney, and the spleen of the living organism, the method comprising the steps of:
a) administering the marker substance to a living organism by inhalation,
b) determining a concentration of the marker substance in exhaled air which is exhaled by the living organism at a first time point, wherein the first time point is less than 30 seconds after the end of inhalation of the marker substance,
c) determining the concentration of the marker substance in the exhaled air which is exhaled by the living organism at a second time point after the first time point, wherein a difference between the first time point and the second time point is between 1 minute and 30 minutes,
d) determining a difference between the concentration of the marker substance determined at the first time point and the concentration of the marker substance determined at the second time point,
e) indirectly observing a metabolization dynamics of the marker substance in at least one of the liver, the kidney, and the spleen by a decrease of the concentration of the marker substance in the exhaled air between the first time point and the second time point.

4. Use according to claim 3, **characterized in that** the terpene is at least one of the group consisting of α-pinene, β-pinene and γ-pinene.

5. Use according to claim 3, **characterized in that** the terpene is limonene.

6. Substance chosen from the group consisting of octane, furan-2-ylmethanethiol, 1,2-diazine, 1,3-diazine, 1,4-diazine, a terpene, 2-pentanone, and 4-hydroxynonenal for use as marker substance to be administered to a living organism by inhalation in a diagnostic method for quantitatively determining the function of at least one of the liver, the kidney, and the spleen of the living organism or for diagnosing a severity of a disease of at least one of the liver, the kidney, and the spleen of the living organism, the method comprising the steps of:
a) administering the marker substance to a living organism by inhalation,
b) determining a concentration of the marker substance in exhaled air which is exhaled by the living organism at a first time point, wherein the first time point is less than 30 seconds after the end of inhalation of the marker substance,
c) determining the concentration of the marker substance in the exhaled air which is exhaled by the living organism at a second time point after the first time point, wherein a difference between the first time point and the second time point is between 1 minute and 30 minutes,
d) determining a difference between the concentration of the marker substance determined at the first time point and the concentration of the marker substance determined at the second time point,
e) indirectly observing a metabolization dynamics of the marker substance in at least one of the liver, the kidney, and the spleen by a decrease of the concentration of the marker substance in the exhaled air between the first time point and the second time point.

7. Substance for use according to claim 6, **characterized in that** the terpene is at least one of the group consisting of α-pinene, β-pinene and γ-pinene.

8. Substance for use according to claim 6, **characterized in that** the terpene is limonene.

## Patentansprüche

1. Verfahren zur Bereitstellung von Originaldaten, die zur anschließenden quantitativen Ermittlung der Funktion eines Organs eines lebenden Organismus oder zur anschließenden Diagnose einer Schwere einer Erkrankung eines Organs eines lebenden Organismus verwendet werden können, wobei das Verfahren die folgenden Schritte umfasst:
a) Verabreichen einer Markersubstanz an einen lebenden Organismus durch Einatmen, wobei die Markersubstanz einen Dampfdruck über 0,01 mmHg bei 37 °C hat und mindestens eine Substanz aus der aus Octan, Furan-2-ylmethanthiol, 1,2-Diazin, 1,3-Diazin, 1,4-Diazin, einem Terpen, 2-Pentanon und 4-Hydroxynonenal bestehenden Gruppe ist,
b) Ermitteln einer Konzentration der Markersubstanz in ausgeatmeter Luft, die zu einem ersten Zeitpunkt von dem lebenden Organismus ausgeatmet wird, wobei der erste Zeitpunkt weniger als 30 Sekunden nach dem Ende des Einatmens der Markersubstanz liegt,
c) Ermitteln der Konzentration der Markersubstanz in der ausgeatmeten Luft, die zu einem zweiten Zeitpunkt nach dem ersten Zeitpunkt von dem lebenden Organismus ausgeatmet wird, wobei eine Differenz zwischen dem ersten Zeitpunkt und dem zweiten Zeitpunkt zwischen 1 Minute und 30 Minuten beträgt,
d) Ermitteln einer Differenz zwischen der zu dem ersten Zeitpunkt ermittelten Konzentration der Markersubstanz und der zu dem zweiten Zeitpunkt ermittelten Konzentration der Markersubstanz,
e) indirektes Beobachten einer Metabolisierungsdynamik der Markersubstanz in mindestens einem von der Leber, der Niere und der Milz durch eine Abnahme der Konzentration der Markersubstanz in der ausgeatmeten Luft zwischen dem ersten Zeitpunkt und dem zweiten Zeitpunkt.

2. Verfahren nach Anspruch 1, **dadurch gekennzeichnet, dass** das Terpen mindestens eines aus der aus Limonen, α-Pinen, β-Pinen und γ-Pinen bestehenden Gruppe ist.

3. Verwendung mindestens einer Substanz, die aus der aus Octan, Furan-2-ylmethanthiol, 1,2-Diazin, 1,3-Diazin, 1,4-Diazin, einem Terpen, 2-Pentanon und 4-Hydroxynonenal bestehenden Gruppe ausgewählt ist, als Markersubstanz, die durch Einatmen an einen lebenden Organismus zu verabreichen ist, in einem Verfahren zur Bereitstellung von Originaldaten, die i) zur anschließenden quantitativen Ermittlung der Funktion mindestens eines von der Leber, der Niere und der Milz des lebenden Organismus oder ii) zur anschließenden Diagnose einer Schwere einer Erkrankung mindestens eines von der Leber, der Niere und der Milz des lebenden Organismus verwendet werden können, wobei das Verfahren die folgenden Schritte umfasst:
a) Verabreichen der Markersubstanz an einen lebenden Organismus durch Einatmen,
b) Ermitteln einer Konzentration der Markersubstanz in ausgeatmeter Luft, die zu einem ersten Zeitpunkt von dem lebenden Organismus ausgeatmet wird, wobei der erste Zeitpunkt weniger als 30 Sekunden nach dem Ende des Einatmens der Markersubstanz liegt,
c) Ermitteln der Konzentration der Markersubstanz in der ausgeatmeten Luft, die zu einem zweiten Zeitpunkt nach dem ersten Zeitpunkt von dem lebenden Organismus ausgeatmet wird, wobei eine Differenz zwischen dem ersten Zeitpunkt und dem zweiten Zeitpunkt zwischen 1 Minute und 30 Minuten beträgt,
d) Ermitteln einer Differenz zwischen der zu dem ersten Zeitpunkt ermittelten Konzentration der Markersubstanz und der zu dem zweiten Zeitpunkt ermittelten Konzentration der Markersubstanz,
e) indirektes Beobachten einer Metabolisierungsdynamik der Markersubstanz in mindestens einem von der Leber, der Niere und der Milz durch eine Abnahme der Konzentration der Markersubstanz in der ausgeatmeten Luft zwischen dem ersten Zeitpunkt und dem zweiten Zeitpunkt.

4. Verwendung nach Anspruch 3, **dadurch gekennzeichnet, dass** das Terpen mindestens eines aus der aus einem α-Pinen, β-Pinen und γ-Pinen bestehenden Gruppe ist.

5. Verwendung nach Anspruch 3, **dadurch gekennzeichnet, dass** es sich bei dem Terpen um Limonen handelt.

6. Substanz, die aus der aus Octan, Furan-2-ylmethanthiol, 1,2-Diazin, 1,3-Diazin, 1,4-Diazin, einem Terpen, 2-Pentanon und 4-Hydroxynonenal bestehenden Gruppe ausgewählt ist, zur Verwendung als Markersubstanz, die bei einem Diagnoseverfahren zur quantitativen Ermittlung der Funktion mindestens eines von der Leber, der Niere und der Milz des lebenden Organismus oder zur Diagnose einer Schwere einer Erkrankung von mindestens einem von der Leber, der Niere und der Milz des lebenden Organismus durch Einatmen an einen lebenden Organismus zu verabreichen ist, wobei das Verfahren die folgenden Schritte umfasst:
a) Verabreichen der Markersubstanz an einen lebenden Organismus durch Einatmen,
b) Ermitteln einer Konzentration der Markersubstanz in ausgeatmeter Luft, die zu einem ersten Zeitpunkt von dem lebenden Organismus ausgeatmet wird, wobei der erste Zeitpunkt weniger als 30 Sekunden nach dem Ende des Einatmens der Markersubstanz liegt,
c) Ermitteln der Konzentration der Markersubstanz in der ausgeatmeten Luft, die zu einem zweiten Zeitpunkt nach dem ersten Zeitpunkt von dem lebenden Organismus ausgeatmet wird, wobei eine Differenz zwischen dem ersten Zeitpunkt und dem zweiten Zeitpunkt zwischen 1 Minute und 30 Minuten beträgt,
d) Ermitteln einer Differenz zwischen der zu dem ersten Zeitpunkt ermittelten Konzentration der Markersubstanz und der zu dem zweiten Zeitpunkt ermittelten Konzentration der Markersubstanz,
e) indirektes Beobachten einer Metabolisierungsdynamik der Markersubstanz in mindestens einem von der Leber, der Niere und der Milz durch eine Abnahme der Konzentration der Markersubstanz in der ausgeatmeten Luft zwischen dem ersten Zeitpunkt und dem zweiten Zeitpunkt.

7. Substanz zur Verwendung nach Anspruch 6, **dadurch gekennzeichnet, dass** das Terpen mindestens eines aus der aus einem α-Pinen, **-**Pinen und γ-Pinen bestehenden Gruppe ist.

8. Substanz zur Verwendung nach Anspruch 6, **dadurch gekennzeichnet, dass** es sich bei dem Terpen um Limonen handelt.

## Revendications

1. Procédé pour fournir des données originales qui peuvent être utilisées pour déterminer ultérieurement, de manière quantitative, la fonction d'un organe d'un organisme vivant ou pour diagnostiquer ultérieurement une gravité d'une maladie d'un organe d'un organisme vivant, ledit procédé comprenant les étapes suivantes :
a) l'administration d'une substance de marquage à un organisme vivant par inhalation, la substance de marquage ayant une pression de vapeur supérieure à 0,01 mmHg à 37 °C et étant au moins une substance choisie parmi le groupe constitué de l'octane, du furane-2-ylméthanethiol, de la 1,2-diazine, la 1,3-diazine, la 1,4-diazine, un terpène, la 2-pentanone et le 4-hydroxynonénal,
b) déterminer une concentration de la substance de marquage dans l'air expiré qui est expiré par l'organisme vivant à un premier instant, ce premier instant intervenant moins de 30 secondes après la fin de l'inhalation de la substance de marquage,
c) déterminer la concentration de la substance de marquage dans l'air expiré qui est expiré par l'organisme vivant à un deuxième instant après le premier instant, la différence entre le premier instant et le deuxième instant étant comprise entre 1 minute et 30 minutes,
d) déterminer une différence entre la concentration de la substance de marquage déterminée au premier instant et la concentration de la substance de marquage déterminée au deuxième instant,
e) observer indirectement une dynamique de métabolisation de la substance de marquage dans au moins l'un du foie, des reins et de la rate par une diminution de la concentration de la substance de marquage dans l'air expiré entre le premier instant et le deuxième instant.

2. Procédé selon la revendication 1, **caractérisé en ce que** le terpène est au moins l'un des composés du groupe constitué par le limonène, l'α-pinène, le β-pinène et le γ-pinène.

3. Utilisation d'au moins une substance choisie parmi le groupe constitué de l'octane, du furane-2-ylméthane-thiol, de la 1,2-diazine, la 1,3-diazine, la 1,4-diazine, un terpène, la 2-pentanone et le 4-hydroxynonénal, en tant que substance de marquage à administrer à un organisme vivant par inhalation dans un procédé pour fournir des données originales qui peuvent être utilisées i) pour déterminer ultérieurement, de manière quantitative, la fonction d'au moins l'un du foie, des reins et de la rate de l'organisme vivant, ou ii) pour diagnostiquer ultérieurement une gravité d'une maladie touchant d'au moins l'un parmi le foie, les reins et la rate de l'organisme vivant, le procédé comprenant les étapes suivantes :
a) l'administration de la substance de marquage à un organisme vivant par inhalation,
b) déterminer une concentration de la substance de marquage dans l'air expiré qui est expiré par l'organisme vivant à un premier instant, ce premier instant intervenant moins de 30 secondes après la fin de l'inhalation de la substance de marquage,
c) déterminer la concentration de la substance de marquage dans l'air expiré qui est expiré par l'organisme vivant à un deuxième instant après le premier instant, la différence entre le premier instant et le deuxième instant étant comprise entre 1 minute et 30 minutes,
d) déterminer une différence entre la concentration de la substance de marquage déterminée au premier instant et la concentration de la substance de marquage déterminée au deuxième instant,
e) observer indirectement une dynamique de métabolisation de la substance de marquage dans au moins l'un du foie, des reins et de la rate par une diminution de la concentration de la substance de marquage dans l'air expiré entre le premier instant et le deuxième instant.

4. Utilisation selon la revendication 3, **caractérisée en ce que** le terpène est au moins l'un des composés du groupe constitué par l'α-pinène, le β-pinène et le γ-pinène.

5. Utilisation selon la revendication 3, **caractérisée en ce que** le terpène est le limonène.

6. Substance choisie parmi le groupe constitué de l'octane, du furane-2-ylméthane-thiol, de la 1,2-diazine, la 1,3-diazine, la 1,4-diazine, un terpène, la 2-pentanone et le 4-hydroxynonénal, destinée à être utilisée comme substance de marquage à administrer à un organisme vivant par inhalation dans un procédé diagnostique pour déterminer quantitativement la fonction d'au moins l'un du foie, des reins et de la rate de l'organisme vivant, ou pour diagnostiquer une gravité d'une maladie d'au moins l'un du foie, des reins et de la rate de l'organisme vivant, le procédé comprenant les étapes suivantes :
a) l'administration de la substance de marquage à un organisme vivant par inhalation,
b) déterminer une concentration de la substance de marquage dans l'air expiré qui est expiré par l'organisme vivant à un premier instant, ce premier instant intervenant moins de 30 secondes après la fin de l'inhalation de la substance de marquage,
c) déterminer la concentration de la substance de marquage dans l'air expiré qui est expiré par l'organisme vivant à un deuxième instant postérieur au premier instant, la différence entre le premier instant et le deuxième instant étant comprise entre 1 minute et 30 minutes,
d) déterminer une différence entre la concentration de la substance de marquage déterminée au premier instant et la concentration de la substance de marquage déterminée au deuxième instant,
e) observer indirectement une dynamique de métabolisation de la substance de marquage dans au moins l'un du foie, des reins et de la rate par une diminution de la concentration de la substance de marquage dans l'air expiré entre le premier instant et le deuxième instant.

7. Substance destinée à être utilisée selon la revendication 6, **caractérisée en ce que** le terpène est au moins l'un des composés du groupe constitué par l'α-pinène, le β-pinène et le γ-pinène.

8. Substance destinée à être utilisée selon la revendication 6, **caractérisée en ce que** le terpène est le limonène.
